# DEMANDE DE BREVET EUROPEEN

(11) **EP 0 586 764 A1**
(43) Date de publication de la demande: **16.03.1994**
(21) Numéro de dépôt: 92402399.7
(22) Date de dépôt: 03.09.1992
(51) Int. Cl.: A61K 7/06, A61K 7/08, A61K 7/07

(54) **Produit capillaire sec et son procédé de fabrication**

(71) Demandeur: Leymond, Jean Jack, F-75020 Paris (FR); Garraud, Jacques, F-75001 Paris (FR); Desbois, Gilles, F-95630 Meriel (FR)
(72) Inventeur: Leymond, Jean Jack, F-75020 Paris (FR); Garraud, Jacques, F-75001 Paris (FR); Desbois, Gilles, F-95630 Meriel (FR)
(74) Mandataire: Coutel, Jean-Claude

(57) **Abrégé**

L'invention concerne un produit capillaire sec qui est caractérisé en ce qu'il est formé par une poudre qui est à appliquer directement sur la chevelure et/ou le cuir chevelu et qui est constituée par des filaments de polyamide finement divisés, colorés et présentant des propriétés électrostatiques de répulsion mutuelle. Elle concerne également un procédé de fabrication de ce produit suivant lequel on divise finement des fils de polyamide pour obtenir la poudre, on mélange à sec cette poudre avec au moins un colorant en poudre, et on fait passer la poudre colorée dans une cuve électrostatique.

## Description

La présente invention est relative à un produit capillaire sec et elle a pour but de fournir un produit de ce genre, à utiliser par des professionnels de la coiffure ou par le grand public, ce produit présentant les propriétés qui consistent, sans altérer les cheveux, à les épaissir et à leur donner du volume, ainsi qu'à masquer les calvities naissantes et à diminuer la séborrhée.

A cet effet, le produit capillaire sec selon l'invention est caractérisé en ce qu'il est formé par une poudre qui est à appliquer directement sur la chevelure et/ou le cuir chevelu et qui est constituée par des filaments de polyamide finement divisés, colorés et présentant des propriétés électrostatiques de répulsion mutuelle.

Les constituants élémentaires colorés du produit capillaire sec en poudre selon l'invention, qu'ils soient appliqués ou déposés sur les cheveux ou sur le cuir chevelu, se superposent sans s'accoler, comme s'ils se repoussaient mutuellement, en donnant ainsi du volume par l'électricité statique qu'ils contiennent.De ce fait, et sans aucune action chimique sur les cheveux ou sur la peau, c'est-à-dire sans les altérer, les constituants élémentaires donnent du gonflant aux cheveux, c'est-à-dire qu'ils les épaississent et leur donnent du volume, qu'ils masquent les calvities naissantes par l'effet combiné du gonflant et du recouvrement du cuir chevelu, et qu'ils diminuent la séborrhée.

Le produit selon l'invention est conditionné sous toute forme appropriée, notamment en flacons ou étuis en matière plastique munis d'un bouchon perforé, et il se présente avantageusement sous diverses teintes, correspondant à la gamme habituelle des couleurs de cheveux.

Les constituants élémentaires du produit selon l'invention, à base de filaments ou de bâtonnets de polyamide, ont une longueur par exemple de 0,6 à 0,7 mm, et ils présentent un diamètre qui est par exemple de l'ordre de 8 à 10 microns. Le produit selon l'invention est utilisé par application directe sur les cheveux et/ou sur le cuir chevelu, cette application étant éventuellement suivie d'un brassage à la main pour une meilleure répartition des constituants élémentaires de la poudre.

L'invention vise également un procédé pour la fabrication du produit capillaire sec ci-dessus qui se présente sous la forme d'une poudre constituée par des filaments finement divisés de polyamide, ces filaments étant colorés et présentant des propriétés électrostatiques de répulsion mutuelle.

Pour fabriquer ce produit, on part de fils de polyamide qui présentent avantageusement un diamètre de l'ordre de 8 à 10 microns. Ces fils sont finement coupés ou divisés dans tout appareil approprié, par exemple un broyeur du commerce, pour donner naissance à des filaments ou bâtonnets qui ont une longueur de l'ordre de 0,6 à 0,7 mm. On procède ensuite à la coloration de cette poudre. Cette coloration s'effectue à sec avec au moins un colorant en poudre. Avantageusement, cette opération de coloration s'effectue en deux temps, à savoir une première étape dans laquelle Je colorant est présent suivant une concentration appropriée, par exemple de l'ordre de 10 %, et une seconde étape dans laquelle on ajoute le produit brut, c'est-à-dire non coloré,suivant une quantité permettant d'atteindre le rapport pondéral produit de base/colorant désiré.

On indiquera ci-après, uniquement à titre illustratif et non pas limitatif, quelques exemples de coloration à partir d'une base constituée par une poudre de filaments de polyamide finement divisés.

A titre d'exemples, on a prévu une coloration en noir, une coloration suivant deux nuances de châtain et une coloration suivant deux nuances de blond.

Les colorants de base sont du noir végétal (carbone végétal), constituant un pigment naturel, et des pigments minéraux jaunes (deux teintes et rouge. Ces pigments minéraux sont des oxydes de fer gras de cosmétique.

| | Noir | Châtain I | Châtain II | Blond I | Blond II |
|---|---|---|---|---|---|
| Base(filaments de polyamide) | 95 | 98,5 | 98,4 | 98,8 | 98,9 |
| Noir Végétal (carbone végétal naturel) | 5 | 0,5 | 0,5 | - | - |
| Pigment jaune I | - | 0,5 | 0,5 | - | - |
| Pigment rouge | - | 0,5 | 0,6 | 0,2 | 0,1 |
| Pigment jaune II | - | - | - | 1,0 | 1,0 |

Au cours de la phase ci-dessus de coloration, les filaments de polyamide présentent déjà, sauf dans le cas de la coloration en noir, des propriétés électrostatiques qui résultent de l'utilisation de l'oxyde de fer.

Suivant une phase préférentielle, qui se déroule immédiatement après la coloration, on peut ajouter à la poudre colorée un adoucissant cationique tensio-actif à base de sels d'ammonium quaternaire ou de sels d'alkyl amine.

La poudre reçoit alors un traitement spécifique qui confère à ses filaments les propriétés électrostatiques indiquées ci-dessus qui donnent un effet de gonflant. Lors de cette phase, on fait passer la poudre dans une cuve électrostatique où l'on fait circuler un courant, ce qui confère aux filaments finement divisés les propriétés électrostatiques souhaitées.

Le produit est alors prêt à être conditionné, de la manière indiquée ci-dessus.

## Revendications

1. Produit capillaire sec, caractérisé en ce qu'il est formé par une poudre qui est à appliquer directement sur la chevelure et/ou le cuir chevelu et qui est constituée par des filaments de polyamide finement divisés, colorés et présentant des propriétés électrostatiques de répulsion mutuelle.

2. Produit selon la Revendication 1, caractérisé en ce que les filaments ont une longueur de 0,6 à 0,7 mm et un diamètre de 8 à 10 microns.

3. Procédé pour la fabrication du produit capillaire sec selon l'une des Revendications 1 et 2, caractérisé en ce qu'il comporte les phases consistant à, successivement :
- a)-diviser finement des fils de polyamide pour obtenir une poudre de filaments,
- b)-mélanger à sec ladite poudre avec au moins un colorant en poudre,
- c)-faire passer la poudre ainsi colorée dans une cuve électrostatique.

4. Procédé selon la Revendication 3, caractérisé en ce que la phase b) de coloration se fait en deux étapes, à savoir une première étape dans laquelle on mélange le produit de base avec au moins un colorant, et une seconde étape dans laquelle on ajoute seulement du produit de base.

5. Procédé selon l'une des Revendications 3 et 4, caractérisé en ce que, entre les phases b et c), est prévue une phase d'addition d'un agent tensio-actif, notamment à base de sels d'ammonium quaternaire ou de sels d'alkyl amine.

6. Procédé selon l'une des Revendications 3 à 5, caractérisé en ce que le colorant appartient au groupe constitué par du noir végétal (carbone végétal naturel) et des pigments minéraux (oxydes de fer gras de cosmétique).
